# DEMANDE DE BREVET EUROPEEN

(11) **EP 1 247 515 A2**
(43) Date de publication de la demande: **09.10.2002**
(21) Numéro de dépôt: 02290766.1
(22) Date de dépôt: 27.03.2002
(51) Int. Cl.: A61K 7/043

(54) **Vernis à ongles photoréticulables exempts de monomères insaturés**

(30) Priorité: 06.04.2001 FR 0104679
(71) Demandeur: L'OREAL, 75008 Paris (FR)
(72) Inventeur: Bernard, Pascale, 94370 Sucy-en-Brie (FR); Mondet, Jean, 93600 Aulnay-sous-Bois (FR)
(74) Mandataire: Casalonga, Axel

(57) **Abrégé**

L'invention concerne une composition de vernis à ongles photoréticulable exempte de monomères réactifs comportant une ou plusieurs doubles liaisons éthyléniques et contenant, dans un milieu physiologiquement acceptable,
a) un ou plusieurs polymères comportant des doubles liaisons éthyléniques, le nombre moyen de doubles liaisons éthyléniques par molécule de polymère étant supérieur à 1, et
b) de 0,1 à 10 % en poids, rapportée au poids total de polymère (a) comportant des doubles liaisons éthyléniques, d'au moins un photoamorceur radicalaire,
ainsi qu'un procédé de revêtement cosmétique d'ongles ou de faux-ongles utilisant une telle composition.

## Description

La présente invention concerne des compositions cosmétiques, en particulier des vernis à ongles, photoréticulables, exemptes de monomères, ainsi qu'un procédé de dépôt d'un revêtement réticulé *in situ* utilisant ces compositions.

Les compositions de vernis à ongles peuvent être employées comme base pour vernis, comme produit de maquillage des ongles, comme composition de finition, encore appelée *top-coat* en terminologie anglosaxonne, à appliquer sur le produit de maquillage des ongles ou bien encore comme produit de soin cosmétique des ongles. Ces compositions peuvent s'appliquer aussi bien sur les ongles d'êtres humains que sur des faux-ongles.

On connaît dans le domaine des vernis à ongles des compositions cosmétiques liquides qui, après dépôt sur l'ongle et sous l'action d'un rayonnement actinique, subissent des réactions de polymérisation et/ou de réticulation *in situ* aboutissant à des réseaux polymériques le plus souvent réticulés. De telles compositions photopolymérisables et/ou photoréticulables sont décrites par exemple dans les brevets CA 1 306 954 et US 5 456 905.

Ces compositions contiennent généralement un ou plusieurs polymères et/ou prépolymères comportant des doubles liaisons éthyléniques polymérisables, un ou plusieurs monomères réactifs à double(s) liaison(s), au moins un photoamorceur, éventuellement un ou plusieurs agents inhibant la polymérisation prématurée des composants, ou encore des agents photosensibilisants permettant de modifier le spectre d'absorption du système de photoamorçage.

Le principal inconvénient de ces compositions réside dans la toxicité des monomères insaturés utilisés. En effet, ces molécules hautement réactives et de faible poids moléculaire diffusent facilement dans les substrats sous-jacents et adjacents où ils réagissent avec les molécules biologiques.

La demanderesse a cherché à résoudre ce problème de toxicité des compositions cosmétiques photoréticulables de l'art antérieur en mettant au point des compositions cosmétiques photoréticulables contenant, comme seuls composants réactifs polymérisables ou réticulables, des composés macromoléculaires, c'est-à-dire des composants réactifs ayant une masse moléculaire suffisante pour empêcher leur diffusion vers les substrats biologiques voisins.

La présente invention a par conséquent pour objet des compositions de vernis à ongles photoréticulables exemptes de monomères réactifs à double(s) liaison(s) éthylénique(s), contenant, dans un milieu physiologiquement acceptable,
- un ou plusieurs polymères comportant des doubles liaisons éthyléniques, le nombre moyen de doubles liaisons éthyléniques par molécule de polymère étant supérieur à 1, et
- de 0,1 à 10 % en poids, rapportée au poids total de polymère comportant des doubles liaisons éthyléniques, d'au moins un photoamorceur radicalaire.

L'invention a également pour objet un procédé de revêtement cosmétique d'ongles ou de faux-ongles utilisant ces compositions.

Comme indiqué ci-dessus, le nombre moyen de doubles liaisons éthyléniques portées par les polymères formant le réseau polymérique réticulé du revêtement durci final doit être supérieur à 1. En effet, un système polymérisable constitué de molécules (monomères, oligomères ou polymères) portant chacune une seule double liaison forme, après réaction de la totalité des doubles liaisons, un système macromoléculaire à chaînes linéaires ou ramifiées mais non pas réticulées. Seule la présence d'une certaine fraction de molécules portant au moins deux doubles liaisons et jouant de ce fait le rôle d'agent de réticulation permet d'obtenir un système polymérique réticulé.

Dans la mise en pratique de la présente invention, le nombre moyen de fonctions par molécule de polymère est de préférence supérieur à 2 et mieux encore supérieur à 3.

Comme expliqué ci-dessus, l'innocuité des compositions cosmétiques photoréticulables de la présente invention est due au fait que les polymères comportant une ou plusieurs doubles liaisons éthyléniques ont une masse moléculaire suffisante pour empêcher leur diffusion vers et dans les substrats biologiques voisins. On entend par masse moléculaire suffisante une masse moléculaire moyenne en poids supérieure ou égale à 500. En particulier, la masse moléculaire moyenne en poids peut aller de 500 à 10 000.
L'innocuité des compositions sera d'autant meilleure que la masse des polymères photoréticulables sera élevée.
Dans un mode de réalisation préféré de l'invention, les polymères photoréticulables ont une masse moléculaire moyenne en poids supérieure ou égale à 1000, notamment allant de 1000 à 10 000, et de préférence allant de 2000 à 10 000.

Les polymères portant des doubles liaisons éthyléniques et ayant une masse molaire appropriée pour la présente invention sont connus dans la technique et disponibles sur le marché.

On peut citer à titre d'exemples de polymères photoréticulables utilisables dans les compositions cosmétiques de la présente invention :
a) les polyesters à insaturation(s) éthylénique(s) :
   Il s'agit d'un groupe de polymères de type polyester présentant une ou plusieurs doubles liaisons éthyléniques, réparties de manière aléatoire dans la chaîne principale du polymère. Ces polyesters insaturés sont obtenus par polycondensation d'un mélange
      - de diacides carboxyliques aliphatiques linéaires ou ramifiés ou cycloaliphatiques comportant notamment de 3 à 50 atomes de carbone, de préférence de 3 à 20 atomes de carbone, tels que l'acide adipique ou l'acide sébacique, de diacides carboxyliques aromatiques ayant notamment de 8 à 50 atomes de carbone, de préférence de 8 à 20 atomes de carbone, tels que les acides phtaliques, notamment l'acide téréphtalique, et/ou de diacides carboxyliques issus de dimères d'acides gras à insaturations éthyléniques tels que les dimères des acides oléique ou linoléique décrits dans la demande EP-A-959 066 (paragraphe [0021]) commercialisés sous les dénominations Pripol® par la société Unichema ou Empol® par la société Henkel, tous ces diacides devant être exempts de doubles liaisons éthyléniques polymérisables,
      - de diols aliphatiques linéaires ou ramifiés ou cycloaliphatiques comportant notamment de 2 à 50 atomes de carbone, de préférence de 2 à 20 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 atomes de carbone tel que le le bisphénol A et le bisphénol B, et/ou de dimères diols issus de la réduction des dimères d'acides gras tels que définis précédemment, et
      - d'un ou de plusieurs diacides carboxyliques ou leurs anhydrides comportant au moins une double liaison éthylénique polymérisable et ayant de 3 à 50 atomes de carbone, de préférence de 3 à 20 atomes de carbone, tels que l'acide maléique, l'acide fumarique ou l'acide itaconique.
b) les polyesters à groupes (meth)acrylate latéraux et/ou terminaux :
   Il s'agit d'un groupe de polymères de type polyester obtenus par polycondensation d'un mélange
      - de diacides carboxyliques aliphatiques linéaires ou ramifiés ou cycloaliphatiques comportant notamment de 3 à 50 atomes de carbone, de préférence de 3 à 20 atomes de carbone, tels que l'acide adipique ou l'acide sébacique, de diacides carboxyliques aromatiques ayant notamment de 8 à 50 atomes de carbone, de préférence de 8 à 20 atomes de carbone, tels que les acides phtaliques, notamment l'acide téréphtalique, et/ou de diacides carboxyliques issus de dimères d'acides gras à insaturation éthyléniques tels que les dimères des acides oléique ou linoléique décrits dans la demande EP-A-959 066 (paragraphe [0021]) commercialisés sous les dénominations Pripol® par la société Unichema ou Empol® par la société Henkel, tous ces diacides devant être exempts de doubles liaisons éthyléniques polymérisables,
      - de diols aliphatiques linéaires ou ramifiés ou cycloaliphatiques comportant notamment de 2 à 50 atomes de carbone, de préférence de 2 à 20 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 atomes de carbone tel que le bisphénol A et le bisphénol B, et
      - d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.
   Ces polyesters diffèrent de ceux décrits ci-dessus sous le point a) par le fait que les doubles liaisons éthyléniques ne sont pas situées dans la chaîne principale mais sur des groupes latéraux ou à l'extrémité des chaînes. Ces doubles liaisons éthyléniques sont celles des groupes (méth)acrylate présents dans le polymère.
   De tels polyesters sont commercialisés par exemple par la société UCB sous les dénominations EBECRYL® (EBECRYL® 450 : masse molaire 1600, en moyenne 6 fonctions acrylate par molécule, EBECRYL® 652 : masse molaire 1500, en moyenne 6 fonctions acrylate par molécule, EBECRYL® 800 : masse molaire 780, en moyenne 4 fonctions acrylate par molécule, EBECRYL® 810 : masse molaire 1000, en moyenne 4 fonctions acrylate par molécule, EBECRYL® 50 000 : masse molaire 1500, en moyenne 6 fonctions acrylate par molécule)
c) les polyuréthannes et/ou polyurées à groupes (méth)acrylate, obtenus par polycondensation
   - de diisocyanates, triisocyanates et/ou polyisocyanates aliphatiques cycloaliphatiques et/ou aromatiques ayant notamment de 4 à 50, de préférence de 4 à 30 atomes de carbone, tels que l'hexaméthylènediisocyanate, l'isophoronediisocyanate, le toluènediisocyanate, le diphénylméthanediisocyanate ou les isocyanurates de formule 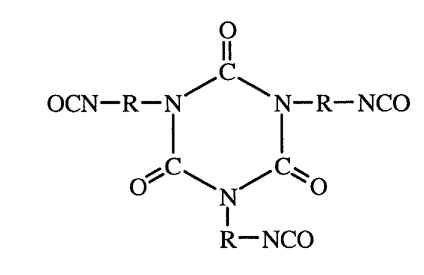 résultant de la trimérisation de 3 molécules de diisocyanates OCN-R-CNO,
   où R est un radical hydrocarboné linéaire, ramifié ou cyclique comportant de 2 à 30 atomes de carbone ;
   - de polyols, notamment de diols, exempts d'insaturations éthyléniques polymérisables, tels que le 1,4-butanediol, l'éthylèneglycol ou le triméthylolpropane, et/ou de polyamines, notamment de diamines, aliphatiques, cycloaliphatiques et/ou aromatiques ayant notamment de 3 à 50 atomes de carbone, telles que l'éthylènediamine ou l'hexaméthylènediamine, et
   - d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.
   De tels polyuréthannes/polyurées à groupes acrylates sont commercialisés par exemple sous la dénomination SR 368 (tris(2-hydroxyéthyl)isocyanuratetriacrylate) ou CRAYNOR® 435 par la société CRAY VALLEY, ou sous la dénomination EBECRYL® par la société UCB (EBECRYL® 210 : masse molaire 1500, 2 fonctions acrylate par molécule, EBECRYL® 230 : masse molaire 5000, 2 fonctions acrylate par molécule, EBECRYL® 270 : masse molaire 1500, 2 fonctions acrylate par molécule, EBECRYL® 8402 : masse molaire 1000, 2 fonctions acrylate par molécule, EBECRYL® 8804 : masse molaire 1300, 2 fonctions acrylate par molécule, EBECRYL® 220 : masse molaire 1000, 6 fonctions acrylate par molécule, EBECRYL® 2220 : masse molaire 1200, 6 fonctions acrylate par molécule, EBECRYL® 1290 : masse molaire 1000, 6 fonctions acrylate par molécule, EBECRYL® 800 : masse molaire 800, 6 fonctions acrylate par molécule).
   On peut également citer les polyuréthannes aliphatiques diacrylate hydrosolubles commercialisés sous les dénominations EBECRYL® 2000, EBECRYL® 2001 et EBECRYL® 2002, et les polyuréthannes diacrylate en dispersion aqueuse commercialisés sous les dénominations commerciales IRR® 390, IRR® 400, IRR® 422 IRR® 424 par la société UCB.
d) les polyéthers à groupes (meth)acrylate obtenus par estérification, par l'acide (méth)acrylique, des groupes hydroxyle terminaux d'homopolymères ou de copolymères d'alkylèneglycols en C₁₋₄, tels que le polyéthylèneglycol, le polypropylèneglycol, les copolymères d'oxyde d'éthylène et d'oxyde de propylène ayant de préférence une masse moléculaire moyenne en poids inférieure à 10 000, le triméthylolpropane polyéthoxylé ou polypropoxylé,.
   Des polyoxyéthylènes-di(méth)acrylate de masse molaire appropriée sont commercialisés par exemple sous les dénominations SR 259, SR 344, SR 610, SR 210, SR 603 et SR 252 par la société CRAY VALLEY ou sous la dénomination EBECRYL® 11 par UCB. Des triacrylates de triméthylolpropane polyéthoxylé sont commercialisés par exemple sous les dénominations SR 454, SR 498, SR 502, SR 9035, SR 415 par la société CRAY VALLEY ou sous la dénomination EBECRYL® 160 par la société UCB. Des triacrylates de triméthylolpropane polypropoxylé sont commercialisés par exemple sous les dénominations SR 492 et SR 501 par la société CRAY VALLEY.
e) les époxyacrylates obtenus par réaction entre
   - au moins un diépoxyde choisi par exemple parmi :
      - l'éther diglycidylique de bisphénol A,
      - une résine diépoxy résultant de la réaction entre l'éther diglycidylique de bisphénol A et l'épichlorhydrine,
      - une résine époxyester à extrémités α,ω-diépoxy résultant de la condensation d'un diacide carboxylique ayant de 3 à 50 atomes de carbone avec un excès stoechiométrique de (i) et/ou (ii), et
      - une résine époxyéther à extrémités α,ω-diépoxy résultant de la condensation d'un diol ayant de 3 à 50 atomes de carbone avec un excès stoechiométrique de (i) et/ou (ii),
      - les huiles naturelles ou synthétiques portant au moins 2 groupes époxyde, telles que l'huile de soja époxydée, l'huile de lin époxydée et l'huile de vernonia époxydée,
      - un polycondensat phénol-formaldéhyde (résine Novolac®), dont les extrémités et/ou les groupes latéraux ont été époxydés,
      et
   - un ou plusieurs acides carboxyliques ou polyacides carboxyliques comportant au moins une double liaison éthylénique en α,β du groupe carboxylique comme l'acide (méth)acrylique ou l'acide crotonique ou les esters d'acide (méth)acrylique et d'un diol ou polyol ayant de 2 à 20 atomes de carbone, de préférence de 2 à 6 atomes de carbone tels que le (méth)acrylate de 2-hydroxyéthyle.
   De tels polymères sont commercialisés par exemple sous les dénominations SR 349, SR 601, CD 541, SR 602, SR 9036, SR 348, CD 540, SR 480, CD 9038 par le société CRAY VALLEY, sous les dénominations EBECRYL® 600 et EBECRYL® 609, EBECRYL® 150, EBECRYL® 860, EBECRYL® 3702 par la société UCB et sous les dénominations PHOTOMER® 3005 et PHOTOMER® 3082 par la société HENKEL.
f) les poly(méth)acrylates d'(alkyle en C₁₋₅₀) comportant au moins deux fonctions à double liaison éthylénique portées par les chaînes hydrocarbonées latérales et/ou terminales.
   De tels copolymères sont commercialisés par exemple sous les dénominations IRR® 375, OTA® 480 et EBECRYL® 2047 par la société UCB.
g) les polyorganosiloxanes à groupes (méth)acrylate ou (méth)acrylamide obtenus respectivement
   - par estérification, p. ex. par l'acide (méth)acrylique, de polyorganosiloxanes, de préférence de polydiméthylsiloxanes (PDMS), portant des groupes hydroxyle terminaux et/ou latéraux,
   - par amidification, par exemple par l'acide (méth)acrylique, de polyorganosiloxanes porteurs de groupes amine primaire ou secondaire latéraux et/ou terminaux.
   Comme PDMS hydroxylés on peut citer en particulier des PDMS comportant au moins deux groupes hydroxyalkyle en C₁₋₆ et les diméthicone-copolyols avec des groupes hydroxyle latéraux ou terminaux.
   Des polydiméthylsiloxanes α,ω-dihydroxylés estérifiables sont commercialisés sous les dénominations TEGOMER® H-Si 2111 et TEGOMER® H-Si 2311 par la société GOLDSCHMIDT. Des polydiméthylsiloxanes α,ω-diacrylate sont disponibles à la société SHIN-ETSU sous les références X-22-164 B et X-22-164C.
   Comme PDMS aminés, on peut citer en particulier des PDMS comportant au moins 2 groupes aminoalkyle en C₁₋₁₀, par exemple la silicone aminée commercialisée sous la dénomination Q2-8220 par la société DOW CORNING.
   Avantageusement, les polymères siliconés de ce groupe sont utilisés en mélange avec un ou plusieurs polymères des autres groupes a) à f) décrits ci-dessus, notamment pour modifier le caractère hydrophobe de la composition finale.
h) les perfluoropolyéthers à groupes acrylate obtenus par estérification, par exemple par l'acide (méth)acrylique, de perfluoropolyéthers portant des groupes hydroxyle latéraux et/ou terminaux.
   De tels perfluoropolyéthers α,ω-diols sont décrits notamment dans EP-A-1057849 et sont commercialisés par la société AUSIMONT sous la dénomination FOMBLIN® Z DIOL.
i) les dendrimères et polymères hyperramifiés portant des groupes terminaux (méth)acrylate ou (méth)acrylamide obtenus respectivement par estérification ou amidification de dendrimères et de polymères hyperramifiés à fonctions terminales hydroxyle ou amino, par de l'acide (méth)acrylique.
   Les dendrimères (du grec *dendron* = arbre) sont des molécules polymères "arborescentes", c'est-à-dire très ramifiées inventées par D. A. Tomalia et son équipe au début des années 90 (Donald A. Tomalia *et al*., Angewandte Chemie, Int. Engl. Ed., vol. 29, n° 2, pages 138 - 175). Il s'agit de structures construites autour d'un motif central généralement polyvalent. Autour de ce motif central, sont enchaînés, selon une structure parfaitement déterminée, des motifs ramifiés d'allongement de chaîne donnant ainsi naissance à des macromolécules symétriques, monodispersées ayant une structure chimique et stéréochimique bien définie. Des dendrimères de type polyamidoamine sont commercialisés par exemple sous la dénomination STARBUST® par la société DENDRITECH.
   Les polymères hyperramifiés sont des polycondensats, généralement de type polyester, polyamide ou polyéthylèneamine, obtenus à partir de monomères multifonctionnels, qui ont une structure arborescente similaire à celle des dendrimères mais beaucoup moins régulière que celle-ci (voir par exemple WO-A-93/17060 et WO 96/12754).
   La société PERSTORP commercialise sous la dénomination BOLTORN® des polyesters hyperramifiés. On trouvera sous la dénomination COMBURST® de la société DENDRITECH des polyéthylèneamines hyperramifiées. Des poly(esteramide) hyperramifiés à extrémités hydroxyle sont commercialisés par la société DSM sous la dénomination HYBRANE®.

Ces dendrimères et polymères hyperramifiés estérifiés ou amidifiés par l'acide acrylique et/ou méthacrylique se distinguent des polymères décrits sous les points a) à h) ci-dessus par le très grand nombre de doubles liaisons éthyléniques présentes. Cette fonctionnalité élevée, le plus souvent supérieure à 5, les rend particulièrement utiles en leur permettant de jouer un rôle de "noeud de réticulation", c'est-à-dire de site de réticulation multiple.
Dans un mode de réalisation préféré de l'invention, on utilisera par conséquent ces polymères dendritiques et hyperramifiés en association avec un ou plusieurs des polymères et/ou oligomères a) à h) ci-dessus.

Comme autres polymères utilisables selon l'invention, on peut également utiliser un polyène associé à un polythiol.

Le polyène est un polymère tel que ceux définis précédemment sous a) à i), ou bien un polymère comportant au moins deux groupes allyliques terminaux et/ou latéraux. Ce denier polymère peut résulter de la polycondensation
- de diols aliphatiques linéaires ou ramifiés ou cycloaliphatiques comportant notamment de 2 à 50 atomes de carbone, de préférence de 2 à 20 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 atomes de carbone tel que le bisphénol A ou le bisphénol B,
- de diacides carboxyliques aliphatiques linéaires ou ramifiés ou cycloaliphatiques comportant notamment de 3 à 50 atomes de carbone, de préférence de 3 à 20 atomes de carbone, tels que l'acide adipique ou l'acide sébacique, de diacides carboxyliques aromatiques ayant notamment de 8 à 50 atomes de carbone, de préférence de 8 à 20 atomes de carbone, tels que les acides phtaliques, notamment l'acide téréphtalique, et/ou de diacides carboxyliques issus de dimères d'acides gras à insaturations éthyléniques, ou de diisocyanates, triisocyanates et/ou polyisocyanates aliphatiques cycloaliphatiques et/ou aromatiques ayant notamment de 4 à 50, de préférence de 4 à 30 atomes de carbone
en présence d'un monoalcool porteur de 2 groupes à insaturation allylique comme l'éther diallylique de triméthylolpropane. Ce monoalcool joue le rôle d'agent de terminaison de chaîne.

Le polythiol est un composé organique oligomère ou polymère portant plus de 2 groupes thiol latéraux ou terminaux. Par exemple, les polythiols résultent de l'estérification d'un polyol, comportant notamment de 4 à 10 atomes de carbone, et d'un monoacide carboxylique à fonction thiol comportant notamment de 2 à 6 atomes decarbone, tel que l'acide thioglycolique ou l'acide 2-mercaptopropionique. Comme polythiol, on peut citer le tris-(β-mercaptopropionate) de triméthylolpropane et le tétrathioglycolate de pentaérythritol.

Les polymères comportant des doubles liaisons éthyléniques décrits ci-dessus représentent généralement de 0,1 à 99 % en poids, et de préférence de 1 à 90 % en poids de la composition cosmétique photoréticulable de la présente invention.

Les photoamorceurs utilisables dans les compositions cosmétiques de la présente invention sont également connus dans la technique et sont décrits, par exemple dans les articles suivants dont le contenu fait partie intégrante de la présente demande : "Les photoinitiateurs dans la réticulation des revêtements", G. Li Bassi, Double Liaison - Chimie des Peintures, n° 361, novembre 1985, pages 34 - 41 ; "Applications industrielles de la polymérisation photoinduite", Henri Strub, L'Actualité Chimique, février 2000, pages 5 - 13 ; et "Photopolymères : considérations théoriques et réaction de prise", Marc, J.M. Abadie, Double Liaison - Chimie des Peintures, n° 435 - 436, 1992, pages 28 - 34.

Ces photoamorceurs englobent
- les α-hydroxycétones, commercialisées par exemple sous les dénominations IRGACURE® 184, 1173, 2959, 149, 1000, 500 et 4265 par la société CIBA,
- les α-aminocétones, commercialisés par exemple sous les dénominations IRGACURE® 907 et 369 par la société CIBA,
- les chloroacétophénones commercialisées par exemple sous les dénominations TRIGONAL® P par la société AKZO et SANDORAY® 1000 par la société SANDOZ,
- les cétones aromatiques commercialisées par exemple sous les dénominations DAITOCURE® par DAINIPPON, UVECRYL® P 36 par UCB, ESACURE® TZT par LAMBERTI, et QUANTACURE® ITX par WARD BLENKINSOP. On peut également citer les thioxanthones (par exemple ULTRACURE® DXT de SHERWIN WILLIAMS) et les quinones (2-éthylanthraquinone de BASF). Ces cétones aromatiques nécessitent le plus souvent la présence d'un composé donneur d'hydrogène tel que les amines tertiaires et plus particulièrement les alcanolamines
- les éthers de benzoïne commercialisés par exemple sous la dénomination ESACURE® EB-3 par la société LAMBERTI et sous la dénomination TRIGONAL® 14 par AKZO,
- les dérivés α-dicarbonyles dont le représentant le plus courant est le benzyldiméthylcétal commercialisé sous la dénomination IRGACURE® 651 par CIBA. D'autres produits commerciaux sont commercialisés par la société LAMBERTI sous la dénomination ESACURE® KBO et par la société WARD BENKINSOP sous la dénomination QUANTACURE® PDO.
- les oxydes d'acylphosphine, tels que par exemple les oxydes de bis-acylphosphine (BAPO) commercialisés par exemple sous la dénomination IRGACURE® 819, 1700, 1800 et 1850 et DAROCUR® 4265 par la société CIBA.

Un groupe particulier de photoamorceurs utilisables dans les compositions cosmétiques photoréticulables de la présente invention est celui des photoamorceurs copolymérisables. Il s'agit de molécules comportant à la fois un groupe photoamorceur capable d'une scission radicalaire photoinduite et au moins une double liaison éthylénique. Les photoamorceurs de ce groupe présentent l'avantage par rapport aux photoamorceurs classiques énumérés ci-dessus de pouvoir être intégrés, par l'intermédiaire de la double liaison, dans le système macromoléculaire. Cette possibilité diminue la concentration de photoamorceurs résiduels libres n'ayant pas subi de coupure radicalaire photoinduite et améliore par conséquent l'innocuité des compositions cosmétiques.
On peut citer à titre d'exemples de tels photoamorceurs copolymérisables les dérivés acrylés de benzophénone commercialisés par la société UCB sous les dénominations EBECRYL® P36 et EBECRYL® P37.

Dans un mode de réalisation préféré de l'invention, on utilise des photoamorceurs polymères ou des photoamorceurs fixés sur une molécule de masse molaire élevée. Le choix d'un tel photoamorceur de masse élevée présente le même avantage que la sélection de composants copolymérisables exclusivement polymères, à savoir une meilleure innocuité des compositions cosmétiques photoréticulables due à l'absence de petites molécules très réactives susceptibles de diffuser vers les substrats biologiques voisins. Comme pour les composants copolymérisables, la masse moléculaire moyenne en poids du photoamorceur est de préférence au moins égale à 500.

On peut citer à titre d'exemple l'oligomère d'α-hydroxycétone correspondant à la formule suivante : 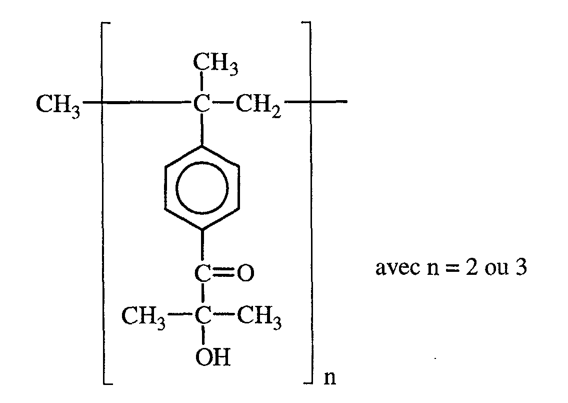 et qui est commercialisé sous les dénominations ESACURE® KIP 150 et ESACURE® KIP EM par la société LAMBERTI.

Le polymère sur lequel est fixé le groupe photoamorceur peut éventuellement comporter une ou plusieurs doubles liaisons éthyléniques permettant éventuellement l'intégration, dans le réseau macromoléculaire, des molécules de photoamorceurs n'ayant pas subi de scission photoinduite.
On peut citer à titre d'exemples de tels photoamorceurs de masse moléculaire élevée portant des doubles liaisons éthyléniques, ceux correspondant aux formules suivantes : 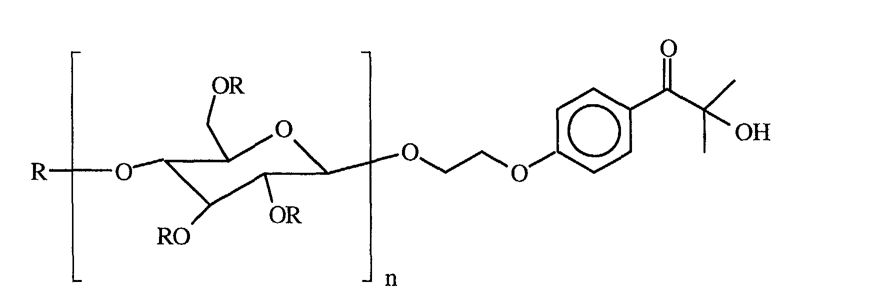 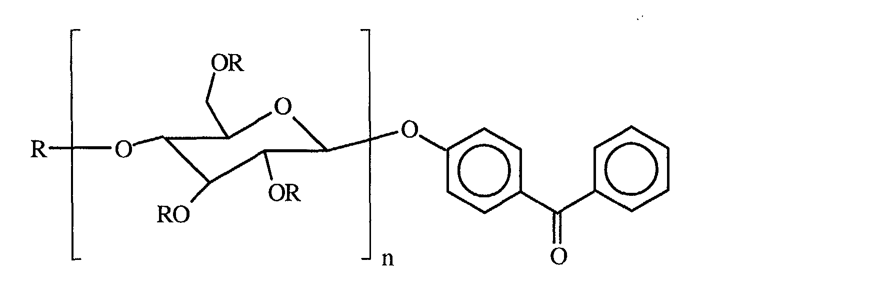 avec n = 1 à 20 R = H ou 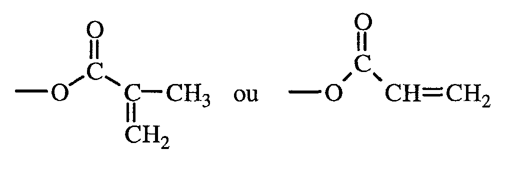 structures décrites dans les articles suivants : S. Knaus, *Pure Appl. Chem.,* A33(7), 869 (1996) ; S. Knaus, *J. Polym. Sci, Part A* = *Polym. Chem.,* 33, 929 (1995) ; et R. Liska, *Rad'Tech Europe* 97, Lyon, F, 1997, Conference Proceedings.

On utilise de préférence dans les compositions cosmétiques photoréticulables de la présente invention un mélange de photoamorceurs absorbant la lumière à différentes longueurs d'ondes. Il est ainsi possible d'adapter le spectre d'absorption des compositions cosmétiques photoréticulables au spectre d'émission des sources de lumière utilisées.

La concentration du ou des photoamorceurs utilisée dépend d'un grand nombre de facteurs comme par exemple de la réactivité des différents composants du mélange, de la présence de pigments ou de colorants, de la densité de réticulation recherchée, de l'intensité de la source lumineuse ou du temps d'exposition.
Pour obtenir des propriétés mécaniques satisfaisantes, la quantité totale de photoamorceur(s) doit être comprise entre 0,1 et 10 % en poids rapportée au poids total de polymère comportant des doubles liaisons éthyléniques, et on utilisera de préférence une quantité comprise entre 0,2 % et 5 % en poids, rapportée au poids total de polymère (a) comportant des doubles liaisons éthyléniques.

Les compositions cosmétiques photoréticulables de la présente invention peuvent contenir un ou plusieurs solvants choisis parmi l'eau et les solvants organiques physiologiquement acceptables parmi lesquels on peut citer
a) les cétones liquides à température ambiante telles que la méthyléthylcétone, la méthylisobutylcétone, la diisobutylcétone, l'isophorone, la cyclohexanone et l'acétone,
b) les alcools liquides à température ambiante tels que l'éthanol, l'isopropanol, le diacétone-alcool, le 2-butoxyéthanol ou le cyclohexanol,
c) les glycols liquides à température ambiante tels que l'éthylèneglycol, le propylèneglycol, le pentylèneglycol et le glycérol,
d) les éthers de propylèneglycol liquides à température ambiante tels que le monométhyléther de propylèneglycol, l'acétate de l'éther monométhylique de propylèneglycol, le mono-n-butyléther de dipropylèneglycol,
e) les esters à courte chaîne (comportant au total de 3 à 8 atomes de carbone) tels que l'acétate d'éthyle, l'acétate de méthyle, l'acétate de propyle, l'acétate de n-butyle et l'acétate d'isopentyle,
f) les alcanes liquides à température ambiante tels que le décane, l'heptane, le dodécane, le cyclohexane,
g) les hydrocarbures aromatiques liquides à température ambiante tels que le toluène et le xylène,
h) les silicones liquides à température ambiante et
i) des mélanges de ceux-ci.

La teneur en solvant dans la composition peut aller de 0,1 % à 80 % en poids, par rapport au poids total de la composition, et de préférence de 1 à 60 % en poids.
Selon un mode particulier de la composition selon l'invention, la composition est exempte de solvant.

Les compositions cosmétiques photoréticulables de la présente invention peuvent contenir en outre des adjuvants et additifs utilisés couramment dans des vernis à ongles, choisis notamment parmi les pigments et colorants, les agents plastifiants, les agents de coalescence, les agents conservateurs, les cires, les agents épaissisants, les parfums, les filtres UV, les actifs cosmétiques pour le soin des ongles, les agents d'étalement, les agents anti-mousses et les agents dispersants.

Bien entendu l'homme du métier veillera à choisir ces éventuels adjuvants et additifs de manière à ce que les propriétés avantageuses des compositions selon l'invention ne soient pas, ou pratiquement pas altérées par l'adjonction envisagée.

Lorsque la composition comprend des pigments et/ou colorants, il convient en particulier d'adapter le spectre d'absorption des pigments et/ou colorants utilisés à celui des photoamorceurs, ou inversement le spectre d'absorption des photoamorceurs à celui des pigments et/ou colorants utilisés, afin d'éviter que ces deux types de composés absorbent la lumière aux mêmes longueurs d'onde. En effet, l'absorption de la lumière par les pigments et/ou colorants rendrait presque totalement inefficaces les photoamorceurs présents au-delà d'une certaine profondeur du revêtement.

La présente invention a également pour objet un procédé de revêtement cosmétique d'ongles naturels ou de faux-ongles.

Ce procédé comprend
- l'application d'une composition cosmétique photoréticulable contenant, dans un milieu physiologiquement acceptable, (a) un ou plusieurs oligomères et/ou polymères comportant des doubles liaisons éthyléniques, le nombre moyen de doubles liaisons éthyléniques par molécule d'oligomère et/ou de polymère étant supérieur à 1, et (b) de 0,1 à 10 % en poids, rapporté au poids total du polymère comportant des doubles liaisons éthyléniques, d'au moins un photoamorceur radicalaire, sur des ongles ou faux-ongles, et
- l'exposition du film déposé à un rayonnement de longueur d'onde approprié pendant un temps suffisant pour induire la réticulation dudit film.

Lorsque la composition comprend un solvant organique volatil ou de l'eau, le film est exposé au rayonnement après évaporation du solvant ou de l'eau.
Dans un mode de réalisation particulier du procédé de la présente invention, le substrat kératinique (ongles, faux-ongles, cils, faux-cils, cheveux et prothèses capillaires) sur lequel est déposée la composition cosmétique photoréticulable est déjà couvert par un premier revêtement cosmétique non réticulable, tels qu'une couche de base ou de vernis à ongles usuel.
La composition cosmétique photoréticulable constitue alors une couche de protection (*top coat*) du premier revêtement cosmétique non réticulable. La composition peut également être déposée seule sur l'ongle pour le protéger. Dans ce mode de réalisation, la composition cosmétique photoréticulable est de préférence non colorée, c'est-à-dire exempte de pigments et de colorants.

Les rayonnements appropriés pour la réticulation des compositions cosmétiques de la présente invention ont une longueur d'onde comprise entre 210 et 600 nm, de préférence entre 250 et 400 nm. On peut également envisager l'utilisation de lasers.
Dans un mode de réalisation préféré de l'invention, on utilise une lampe UV et en particulier une lampe à vapeur de mercure, éventuellement dopée par d'autres éléments, tels que le gallium, permettant de modifier le spectre d'émission de la source lumineuse.

La durée d'exposition du film déposé au rayonnement dépend de différents facteurs tels que la nature chimique et la concentration des composants réactifs ou encore de la densité de réticulation recherchée.
Pour les vernis à ongles, on cherchera généralement à obtenir des résultats satisfaisants pour un temps d'exposition compris entre 1 et 20 minutes, de préférence entre 2 et 10 minutes.

Ce temps de durcissement ne correspond de préférence pas à la réticulation totale du système, c'est-à-dire à la réaction de la totalité des doubles liaisons présentes. Pour garantir une bonne tenue des vernis à ongles dans le temps, il est intéressant d'obtenir à la fois une densité de réticulation importante en surface du film assurant une bonne résistance mécanique, et une faible réticulation au niveau de l'interface revêtement-substrat indispensable à la bonne adhérence du film déposé au substrat.

### Exemple 1

On a préparé un vernis à ongles photoréticulable ayant la composition suivante :

| | |
|---|---|
| polyester à groupements acrylate vendu sous la dénomination EBECRYL® 83 par la société UCB | 96,5 g |
| photoamorceur (IRGACURE® 500, CIBA) | 3,5 g |

On applique la composition sur les ongles sous forme d'un film d'épaisseur d'environ 50 µm et l'on irradie ce film pendant 5 minutes avec un rayonnement UV émis par une lampe "Philips Original UVA" commercialisée par la société Philips dont le domaine de longueur d'onde efficace va de 340 à 365 nm. La lampe est placée à une distance d'environ 50 cm des ongles et pendant la durée d'exposition, on incline les mains alternativement dans un sens et dans l'autre de manière à répartir le rayonnement uniformément sur toute la surface des ongles.
On obtient ainsi un film sec au toucher, non collant, brillant et dur.

### Exemple 2

On procède de la manière décrite dans l'Exemple 1 en utilisant la composition cosmétique suivante :

| | |
|---|---|
| polyester à groupements acrylate vendu sous la dénomination EBECRYL® 140 par la société UCB | 96,5 g |
| photoamorceur (IRGACURE® 500, CIBA) | 3,5 g |
| pigments | q.s. |

On obtient ainsi un film dur, brillant et non collant.

### Exemple 3

On procède de la manière décrite dans l'Exemple 1 en utilisant la composition cosmétique suivante :

| | |
|---|---|
| polyuréthanne-triacrylate vendu sous la dénomination CRAYNOR® 435 par la société CRAY VALLEY | 95 g |
| photoamorceur (IRGACURE® 500, CIBA) | 3,5 g |
| pigments | q.s. |

On obtient ainsi un film dur, brillant et non collant.

## Revendications

1. Composition de vernis à ongles photoréticulable contenant, dans un milieu physiologiquement acceptable,
a) un ou plusieurs polymères comportant des doubles liaisons éthyléniques, le nombre moyen de doubles liaisons éthyléniques par molécule de polymère étant supérieur à 1, et
b) de 0,1 à 10 % en poids, rapportée au poids total de polymère (a) comportant des doubles liaisons éthyléniques, d'au moins un photoamorceur radicalaire,
**caractérisée par le fait qu'**elle est exempte de monomères réactifs de masse moléculaire inférieure à 500 comportant une ou plusieurs doubles liaisons éthyléniques.

2. Composition de vernis à ongles photoréticulable selon la revendication 1,
**caractérisée par le fait que** le nombre moyen de doubles liaisons éthyléniques par molécule de polymère est supérieur à 2, et de préférence supérieur ou égal à 3.

3. Composition de vernis à ongles photoréticulable selon la revendication 1 ou 2, **caractérisée par le fait que** le ou les polymères sont choisis parmi,
a) les polyesters à insaturation(s) éthylénique(s) obtenus par polycondensation
- de diacides carboxyliques aliphatiques linéaires ou ramifiés ou cycloaliphatiques comportant notamment de 3 à 50 atomes de carbone, de préférence de 3 à 20 atomes de carbone, de diacides carboxyliques aromatiques ayant notamment de 8 à 50 atomes de carbone, de préférence de 8 à 20 atomes de carbone, et/ou de diacides carboxyliques issus de dimères d'acides gras à insaturations éthyléniques, tous ces diacides étant exempts de doubles liaisons éthyléniques polymérisables,
- de diols aliphatiques linéaires ou ramifiés ou cycloaliphatiques comportant notamment de 2 à 50 atomes de carbone, de préférence de 2 à 20 atomes de carbone, de diols aromatiques ayant de 6 à 50 atomes de carbone, de préférence de 6 à 20 atomes de carbone, et/ou de dimères diols issus de la réduction des dimères d'acides gras tels que définis précédemment, et
- d'un ou de plusieurs diacides carboxyliques ou leurs anhydrides comportant au moins une double liaison éthylénique polymérisable et ayant de 3 à 50 atomes de carbone, de préférence de 3 à 20 atomes de carbone,
b) les polyesters à groupes (méth)acrylate latéraux et/ou terminaux obtenus par polycondensation
- de diacides carboxyliques aliphatiques linéaires ou ramifiés ou cycloaliphatiques comportant notamment de 3 à 50 atomes de carbone, de préférence de 3 à 20 atomes de carbone, de diacides carboxyliques aromatiques ayant notamment de 8 à 50 atomes de carbone, de préférence de 8 à 20 atomes de carbone, et/ou de diacides carboxyliques issus de dimères d'acides gras à insaturation éthyléniques, tous ces diacides étant exempts de doubles liaisons éthyléniques polymérisables,
- de diols aliphatiques linéaires ou ramifiés ou cycloaliphatiques comportant notamment de 2 à 50 atomes de carbone, de préférence de 2 à 20 atomes de carbone, de diols aromatiques ayant de 6 à 50 atomes de carbone, de préférence de 6 à 20 atomes de carbone, et
- d'au moins un ester d'acide (méth)acrylique et d'un diol ou polyol ayant de 2 à 20 atomes de carbone, de préférence de 2 à 6 atomes de carbone,
c) les polyuréthannes et/ou polyurées à groupes (méth)acrylate obtenus par polycondensation
- de diisocyanates, triisocyanates et/ou polyisocyanates aliphatiques cycloaliphatiques et/ou aromatiques ayant notamment de 4 à 50, de préférence de 4 à 30 atomes de carbone,
- de polyols, notamment de diols, exempts d'insaturations éthyléniques polymérisables, tels que le 1,4-butanediol, l'éthylèneglycol ou le triméthylolpropane, et/ou de polyamines, notamment de diamines, aliphatiques, cycloaliphatiques et/ou aromatiques ayant notamment de 3 à 50 atomes de carbone, et
- d'au moins un ester d'acide (méth)acrylique et d'un diol ou polyol ayant de 2 à 20 atomes de carbone, de préférence de 2 à 6 atomes de carbone
d) les polyéthers à groupes (méth)acrylate obtenus par estérification, par l'acide (méth)acrylique, des groupes hydroxyle terminaux d'homopolymères ou copolymères d'alkylèneglycols en C₁₋₄,
e) les époxyacrylates obtenus par réaction entre
- au moins un diépoxyde et
- un ou plusieurs acides carboxyliques ou polyacides carboxyliques comportant au moins une double liaison éthylénique en α,β du groupe carboxylique comme l'acide (méth)acrylique, l'acide crotonique comportant au moins une double liaison (méth)acrylique, tels que l'acide (méth)acrylique, ou les esters d'acide (méth)acrylique et d'un diol ou polyol ayant de 2 à 20 atomes de carbone, de préférence de 2 à 6 atomes de carbone.
f) les poly(méth)acrylates d'(alkyle en C₁₋₅₀) comportant au moins deux fonctions à double liaison éthylénique portées par les chaînes hydrocarbonées latérales et/ou terminales,
g) les polyorganosiloxanes à groupes (méth)acrylate ou (méth)acrylamide obtenus respectivement
- par estérification, p. ex. par l'acide (méth)acrylique, de polyorganosiloxanes, de préférence de polydiméthylsiloxanes (PDMS), portant des groupes hydroxyle terminaux et/ou latéraux,
- par amidification, par exemple par l'acide (méth)acrylique, de polyorganosiloxanes porteurs de groupes amine primaire ou secondaire latéraux et/ou terminaux,
h) les perfluoropolyéthers à groupes acrylate obtenus par estérification, par exemple par l'acide (méth)acrylique, de perfluoropolyéthers portant des groupes hydroxyle latéraux et/ou terminaux,
i) les dendrimères et polymères hyperramifiés portant des groupes terminaux (méth)acrylate ou (méth)acrylamide obtenus respectivement par estérification ou amidification de dendrimères et de polymères hyperramifiés à fonctions terminales hydroxyle ou amino, par de l'acide (méth)acrylique.

4. Composition de vernis à ongles photoréticulable selon la revendication 3, **caractérisée par le fait qu'**elle contient un ou plusieurs des polymères (a) à (h) en association avec un ou plusieurs dendrimères et/ou polymères hyperramifiés à groupes (méth)acrylate (i).

5. Composition de vernis à ongles photoréticulable selon la revendication 3, **caractérisée par le fait qu'**elle contient un ou plusieurs des polymères (a) à (h) en association avec un polythiol.

6. Composition de vernis à ongles selon la revendication 1 ou 2, **caractérisée par le fait que** le polymère est un polyène comportant au moins deux groupes allyliques terminaux et/ou latéraux, associé à un polythiol, le polyène comportant au moins deux groupes allyliques terminaux et/ou latéraux étant obtenu par polycondensation
- de diols aliphatiques linéaires ou ramifiés ou cycloaliphatiques comportant notamment de 2 à 50 atomes de carbone, de préférence de 2 à 20 atomes de carbone, de diols aromatiques ayant de 6 à 50 atomes de carbone, de préférence de 6 à 20 atomes de carbone et
- de diacides carboxyliques aliphatiques linéaires ou ramifiés ou cycloaliphatiques comportant notamment de 3 à 50 atomes de carbone, de préférence de 3 à 20 atomes de carbone, de diacides carboxyliques aromatiques ayant notamment de 8 à 50 atomes de carbone, de préférence de 8 à 20 atomes de carbone, et/ou de diacides carboxyliques issus de dimères d'acides gras à insaturations éthyléniques, ou de diisocyanates, triisocyanates et/ou polyisocyanates aliphatiques cycloaliphatiques et/ou aromatiques ayant notamment de 4 à 50, de préférence de 4 à 30 atomes de carbone
en présence d'un monoalcool porteur de 2 groupes à insaturation allylique comme l'éther diallylique de triméthylolpropane.

7. Composition de vernis à ongles photoréticulable selon l'une des revendications précédentes, **caractérisée par le fait que** le polymère a une masse molaire moyenne en poids supérieure à 1000, de préférence comprise entre 2000 et 10 000.

8. Composition de vernis à ongles photoréticulable selon l'une quelconque des revendications précédentes, **caractérisée par le fait que** la concentration des polymères à doubles liaisons éthyléniques est comprise entre 0,1 et 99 % en poids, de préférence entre 1 et 90 % en poids, par rapport à la composition de vernis à ongles photoréticulable.

9. Composition de vernis à ongles photoréticulable selon l'une quelconque des revendications précédentes, **caractérisée par le fait que** le photoamorceur radicalaire est choisis parmi les α-hydroxycétones, les α-aminocétones, les chloroacétophénones, les cétones aromatiques de préférence associées à un composé donneur d'hydrogène, les éthers de benzoïne, les α-dicétones aromatiques et les oxydes d'acylphosphine.

10. Composition de vernis à ongles photoréticulable selon l'une quelconque des revendications 1 à 8, **caractérisée par le fait que** le photoamorceur est choisi parmi les photoamorceurs copolymérisables comportant au moins un groupe photoamorceur et au moins une double liaison éthylénique.

11. Composition de vernis à ongles photoréticulable selon l'une quelconque des revendications 1 à 8 **caractérisée par le fait que** le photoamorceur est un oligomère ou polymère de masse moléculaire moyenne au moins égale à 500, ou un photoamorceur immobilisé sur une molécule de masse moléculaire moyenne au moins égale à 500.

12. Composition de vernis à ongles photoréticulable selon la revendication 11, **caractérisée par le fait que** le photoamorceur oligomère est un oligomère de formule 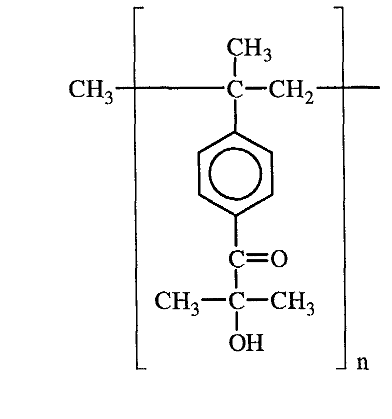 avec n = 2 ou 3

13. Composition de vernis à ongles photoréticulable selon l'une quelconque des revendications précédentes, **caractérisée par le fait que** l'on utilise un mélange de plusieurs photoamorceurs ayant différentes longueurs d'onde d'absorption.

14. Composition de vernis à ongles photoréticulable selon l'une quelconque des revendications précédentes, **caractérisée par le fait que** la quantité totale de photoamorceur est comprise entre 0,2 et 5 % en poids, rapportée au poids total de polymère comportant des doubles liaisons éthyléniques.

15. Composition de vernis à ongles photoréticulable selon l'une quelconque des revendications précédentes, **caractérisée par le fait qu'**elle contient un ou plusieurs solvants choisis parmi l'eau et les solvants organiques physiologiquement acceptables tels que les cétones, alcools, glycols, éthers de propylèneglycols, esters à chaînes courtes, alcanes, hydrocarbures aromatiques et silicones, tous liquides à température ambiante, et des mélanges de ceux-ci.

16. Composition de vernis à ongles photoréticulable selon l'une quelconque des revendications précédentes, **caractérisée par le fait qu'**elle contient en outre des adjuvants et additifs utilisés couramment dans des vernis à ongles, choisis notamment parmi les pigments et colorants, les agents plastifiants, les agents de coalescence, les agents conservateurs, les cires, les agents épaissisants, les parfums, les filtres UV, les actifs cosmétiques pour le soin des ongles, les agents d'étalement, les agents anti-mousses et les agents dispersants.

17. Procédé de revêtement des ongles et faux-ongles comprenant
- l'application d'une composition de vernis à ongles photoréticulable selon l'une quelconque des revendications 1 à 16, sur les ongles ou faux-ongles, et
- l'exposition du film déposé à un rayonnement de longueur d'onde approprié pendant un temps suffisant pour induire la réticulation dudit film.

18. Procédé selon la revendication 17, **caractérisé par le fait que** les ongles ou faux-ongles sont déjà couverts par un premier revêtement cosmétique non réticulable sous rayonnement.

19. Procédé selon l'une des revendications 17 et 18, **caractérisé par le fait que** de rayonnement a une longueur d'onde comprise entre 210 et 600 nm.

20. Procédé selon l'une quelconque des revendications 17 à 19, **caractérisé par le fait que** le temps d'exposition est compris entre 1 et 20 minutes, de préférence entre 2 et 10 minutes.
